(19) **Europäisches Patentamt / European Patent Office / Office européen des brevets**

(11) **EP 3 517 966 B1**

(12) **EUROPEAN PATENT SPECIFICATION**

(45) Date of publication and mention
of the grant of the patent:
**17.11.2021 Bulletin 2021/46**

(21) Application number: **17853475.6**

(22) Date of filing: **22.09.2017**

(51) Int Cl.:
*G01N 33/72* *(2006.01)*        *G01N 33/52* *(2006.01)*
*G01N 33/543* *(2006.01)*        *G01N 21/17* *(2006.01)*

(86) International application number:
**PCT/KR2017/010497**

(87) International publication number:
**WO 2018/056761 (29.03.2018 Gazette 2018/13)**

(54) **REAGENT COMPOSITION FOR MEASURING GLYCATED HEMOGLOBIN AND METHOD FOR MEASURING GLYCATED HEMOGLOBIN USING SAME**

REAGENZZUSAMMENSETZUNG ZUR MESSUNG VON GLYKIERTEM HÄMOGLOBIN UND
VERFAHREN ZUR MESSUNG VON GLYKIERTEM HÄMOGLOBIN DAMIT

COMPOSITION DE RÉACTIF POUR MESURE DE L'HÉMOGLOBINE GLYQUÉE ET PROCÉDÉ DE
MESURE DE L'HÉMOGLOBINE GLYQUÉE L'UTILISANT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.09.2016 KR 20160121689
21.09.2017 KR 20170122083**

(43) Date of publication of application:
**31.07.2019 Bulletin 2019/31**

(73) Proprietor: **DxGen Corp.
Gunpo-si, Gyeonggi-do 15807 (KR)**

(72) Inventors:
• **LEE, Jin Woo
Suwon-si
Gyeonggi-do 16324 (KR)**
• **CHEON, Seon Ah
Seoul 06920 (KR)**

(74) Representative: **Ter Meer Steinmeister & Partner
Patentanwälte mbB
Nymphenburger Straße 4
80335 München (DE)**

(56) References cited:
**WO-A1-2014/033258        KR-A- 20080 023 280
KR-A- 20100 137 851        US-A- 5 919 708
US-A- 5 919 708        US-A1- 2003 073 243
US-A1- 2008 063 868        US-B2- 7 374 943**

• WANG L ET AL: "Watching Silica Nanoparticles
Glow in the Biological World", ANALYTICAL
CHEMISTRY, AMERICAN CHEMICAL SOCIETY,
US, vol. 78, no. 3, 1 February 2006 (2006-02-01),
pages 646-654, XP002554758, ISSN: 0003-2700,
DOI: 10.1021/AC0693619

• WANG, JIASHENG et al.: "Silica-based
Nanocomposites via Reverse Microemulsions:
Classifications, Preparations, and Applications",
Nanoscale, vol. 6, no. 9, 2014, pages 4418-4437,
XP055502107, DOI: doi:10.1039/c3nr06025j

Note: Within nine months of the publication of the mention of the grant of the European patent in the European Patent
Bulletin, any person may give notice to the European Patent Office of opposition to that patent, in accordance with the
Implementing Regulations. Notice of opposition shall not be deemed to have been filed until the opposition fee has been
paid. (Art. 99(1) European Patent Convention).

EP 3 517 966 B1

## Description

## Technical Field

The present invention relates to a reagent composition for measuring glycated hemoglobin to diagnose the presence or absence of diabetes and a method of measuring glycated hemoglobin using the same, and more particularly to a reagent composition for measuring glycated hemoglobin, the composition including a methylene blue-encapsulated silica nanoparticle-boronic acid, and to a method of measuring glycated hemoglobin using the same.

## Background Art

Diabetes is a metabolic disease caused by an abnormality of insulin, which plays a role in regulating blood sugar. It is classified into Type 1 diabetes, which occurs due to insulin deficiency when insulin-producing cells are destroyed due to an abnormality in the immune system, and Type 2 diabetes, caused by a lack of insulin secretion or an ineffective use of insulin, depending on the cause of a disease.

Diabetes is characterized by hyperglycemia, in which the glucose concentration in the blood is elevated. Failure to control blood glucose may lead to complications such as diabetic retinopathy, kidney disease, and foot lesions. Therefore, the importance of blood glucose management for diabetics is increasing.

Conventional diabetes measurement markers use glucose. However, since blood glucose fluctuates greatly before and after a meal, there are problems in that error due to the measurement time and fluctuation due to the condition of the patient may be very evident. Further, measurement

of glucose oxidase, used for glucose measurement, may be vulnerable to environmental influences such as pH or other interfering materials contained in the blood, and hydrogen peroxide may be generated, thus affecting enzyme activity.

Therefore, recently, glycated hemoglobin (HbA1c) has been used as a biomarker that more accurately and stably determines a blood glucose level compared to glucose. Once glycated hemoglobin is generated, it is stable until erythrocytes disappear. Therefore, since glycated hemoglobin may be used as an indicator for showing the mean blood glucose level over 2 to 3 months, it is used to diagnose and investigate the progress of diabetes treatment in practice. However, there is a problem in that a glycated-hemoglobin measurement method is not suited to patients of some diseases that make it difficult to maintain constant blood glucose, as in terminal chronic renal failure or patients with erythrocyte abnormalities.

In order to diagnose diabetes using glycated hemoglobin, it is necessary to judge the amount of glycated hemoglobin relative to the amount of total hemoglobin instead of the absolute value of glycated hemoglobin, because the amounts of total hemoglobin of individuals are different depending on sex, age, and blood-related diseases. Therefore, in order to use glycated hemoglobin as a diagnostic indicator for diabetes, a technique capable of distinguishing the amount of total hemoglobin and the amount of glycated hemoglobin is necessary.

Hemoglobin includes heme, in the center of which iron ions are coordinated, and globin proteins. The hemoglobin has an absorption wavelength of 400 to 600 nm, which varies slightly depending on chemical modification such as the binding of hemoglobin to oxygen and the methylation thereof. Among the absorption wavelengths, an absorption wavelength of 430 nm is a common characteristic of glycated hemoglobin and general hemoglobin, so a measurement thereof may be used to indicate the amount of total hemoglobin. Accordingly, it is necessary to selectively label only glycated hemoglobin in order to distinguish the same. For this purpose, a boronic acid-affinity method is used. This is a method in which a coloring dye or a fluorescent material is bound to boronic acid for recognizing cis-diol at the glucose site of glycated hemoglobin and is then used as a label. The method is excellent in sensitivity and stability, like a reaction adopting an enzyme or an antigen-antibody reaction.

U.S. Patent Nos. 5631364 and 7374943 and International Patent No. 2014-033258 disclose a method that includes reacting a dye-binding boronic acid derivative with a glycated hemoglobin in the blood, loading the resultant substance on a cartridge including a porous filter paper, performing washing, and measuring the reflectances (%) of total hemoglobin and dye-binding glycated hemoglobin, thereby determining the ratios of the two materials.

However, there are problems in that boronic acid derivatives conjugated with a dye are vulnerable to light and heat because they are directly exposed to the external environment and in that a synthesis process thereof is complicated. Most organic-based dye molecules have a problem in that the coloring amount and coloring wavelength are changed or in that light is sometimes extinguished thereby depending on the pH or polarity of the reaction system and the surrounding environment, such as other impurities. Accordingly, there is a need for a method for minimizing the effect of the same.

Meanwhile, European Patent No. 2444803 and Korean Patent No. 1128037 disclose a method of enabling boronic acid derivatives to bind to beads and a method of measuring glycated hemoglobin by directly loading a reacted sample on a cartridge including a porous filter paper. Although the synthesis of beads and boronic acid derivatives is relatively simple, since the same wavelength region of hemoglobin and glycated hemoglobin is measured, total hemoglobin is first measured, and the number of hemoglobin reacted with the bead-boronic acid derivative is measured. In order to remove normal hemoglobin, it is required to perform a cumbersome process for measuring the remaining glycated hemoglobin after washing. Further, since the same wavelength range is measured, caution must be taken to wash away the normal hemoglobin.

As a result of efforts made to solve the above problems, the inventors of the present invention have found that when using boronic acid conjugated with methylene blue-encapsulated silica nanoparticles having different absorption spectra from hemoglobin or modified hemoglobin, the amounts of hemoglobin and glycated hemoglobin are capable of being measured quickly and accurately in a simple and stable manner using an optical instrument, whereby the present invention has been completed.

**Disclosure**

**Technical Problem**

An object of the present invention is to provide a reagent composition for measuring glycated hemoglobin to simply and accurately diagnose the presence or absence of diabetes, and a method of measuring glycated hemoglobin using the same. The invention is set out in the appended set of claims.

**Technical Solution**

In order to accomplish the above object, the present invention provides a reagent composition for measuring glycated hemoglobin. The reagent composition includes ① a hemolytic liquid and ② a "methylene blue-encapsulated silica nanoparticle-boronic acid" specifically binding to the glycated hemoglobin.

The present invention also provides a method of measuring glycated hemoglobin. The method includes (a) introducing a blood sample into a reagent composition for measuring the glycated hemoglobin, the composition including ① a hemolytic liquid and ② a "methylene blue-encapsulated silica nanoparticle-boronic acid" specifically binding to the glycated hemoglobin, followed by hemolysis and reaction, (b) injecting a reactant into an absorption pad of a cartridge, followed by washing with a washing liquid, (c) measuring an optical reflectance of the absorption pad using an optical instrument to measure the amounts of total hemoglobin and glycated hemoglobin, and (d) calculating a ratio of the glycated hemoglobin on the basis of the measured amounts of the total hemoglobin and the glycated hemoglobin.

In the present invention, the hemolytic liquid is selected from the group consisting of TRIS, HEPES, TES, MOPS, and PIPES.

In the present invention, the dye encapsulated in silica nanoparticles is methylene blue.

In the present invention, "methylene blue-encapsulated silica nanoparticles" are manufactured by adding a dye and silica to a mixture solution of water and a surfactant or a mixture solution of water and an organic solvent, followed by agitation and addition of a basic catalyst, and specifically bind to the glycated hemoglobin.

In the present invention, the diameter of "methylene blue-encapsulated silica nanoparticles" is 10 to 500 nm.

In the present invention, the "methylene blue-encapsulated silica nanoparticle-boronic acid" is manufactured by aminating "methylene blue-encapsulated silica nanoparticles", followed by conjugation with 4-carboxylicphenyl boronic acid (CPBA), or by carboxylating or aldehyding the "methylene blue-encapsulated silica nanoparticles", followed by conjugation with 3-aminophenylboronic acid (APBA).

In the present invention, the optical instrument radiates a wavelength for measuring the total hemoglobin and a wavelength for measuring the glycated hemoglobin binding to the "methylene blue-encapsulated silica nanoparticle-boronic acid" as light sources, thus measuring the optical reflectance.

In the present invention, in the method of measuring the glycated hemoglobin, diabetes is diagnosed according to the ratio of the glycated hemoglobin.

**Advantageous Effects**

In a reagent composition for measuring glycated hemoglobin according to the present invention, since a dye is encapsulated in silica nanoparticles, the inherent absorption wavelength of the dye is not affected by pH and the composition has excellent stability even when stored for one month or more. A plurality of dye molecules is encapsulated in a single silica nanoparticle, so that the amount of light absorbed by one particle is larger than that absorbed by one dye molecule. Accordingly, it is possible to accurately measure the amount of glycated hemoglobin in the blood, which conventionally has a low detection limit.

**Description of Drawings**

FIG. 1 is a view showing a method of manufacturing "dye-encapsulated silica nanoparticles" according to an embodiment of the present invention;

FIG. 2 shows the structure of a "dye-encapsulated silica nanoparticle-boronic acid" manufactured according to the embodiment of the present invention;

FIG. 3 is a view showing a binding reaction of the "dye-encapsulated silica nanoparticle-boronic acid" and glycated hemoglobin according to the embodiment of the present invention;

FIG. 4 is a flowchart showing a method of measuring glycated hemoglobin using a reagent composition for measuring glycated hemoglobin and an optical instrument of the present invention;

FIG. 5 is a view showing a radiation method of the optical instrument according to the embodiment of the present invention;

FIG. 6 is a graph showing the similarity between absorbances depending on the absorption wavelength of the "dye-encapsulated silica nanoparticle-boronic acid" manufactured according to the embodiment of the present invention and the absorption wavelength of each of the dyes that are used;

FIG. 7 is a graph showing (A) the shape image of the "dye-encapsulated silica nanoparticle-boronic acid" manufactured according to the embodiment of the present invention, measured using a scanning electron microscope, and (B) the size thereof, analyzed using a dynamic light-scattering method; and

FIG. 8 is a graph showing the reflectance value of the "dye-encapsulated silica nanoparticle-boronic acid" depending on the glycated hemoglobin concentration of a blood sample, the concentration of which is known.

**Best Mode**

In the present invention, the intention is to confirm that when using boronic acid conjugated with methylene blue-encapsulated silica nanoparticles having absorption spectra different from hemoglobin or modified hemoglobin, the amounts of hemoglobin and glycated hemoglobin are capable of being measured accurately in a simple and stable manner using an optical instrument.

In the present invention, a "methylene blue-encapsulated silica nanoparticle-boronic acid" specifically binding to glycated hemoglobin was manufactured, and a hemolytic liquid was added thereto, thus manufacturing a reagent composition for measuring glycated hemoglobin. Next, a blood sample was added to the manufactured reagent composition to perform hemolysis and reaction, and was then injected into an absorption pad, followed by washing. The optical reflectance of the absorption pad was measured using the optical instrument in order to measure the amounts of total hemoglobin and glycated hemoglobin. As a result, it was confirmed that it was possible to diagnose diabetes simply and quickly using the ratio of glycated hemoglobin.

That is, a blue-colored methylene blue dye was encapsulated in silica nanoparticles, the hydroxyl group (-OH) on the surface thereof was substituted with a primary amine group, and 4-carboxylicphenyl-boronic acid (CPBA), which is a glycated hemoglobin-binding material, was fixed to the surface thereof, thus manufacturing a "methylene blue-encapsulated silica nanoparticle-boronic acid". The methylene blue-encapsulated silica nanoparticle-boronic acid was mixed with the hemolytic liquid for hemolysis of the blood sample, thus manufacturing a reagent composition for measuring glycated hemoglobin. Next, the blood sample was added to the manufactured reagent composition to perform hemolysis and reaction, and was then injected into the absorption pad, followed by washing. Red (430 nm) and blue (650 to 700 nm) light sources were radiated on the absorption pad using the optical instrument to thus measure the optical reflectance of total hemoglobin and glycated hemoglobin labeled with the dye. Thereby, it could be confirmed that the ratio of glycated hemoglobin was capable of being measured simply and quickly.

Therefore, the present invention relates to a reagent composition for measuring glycated hemoglobin, the composition including ① a hemolytic liquid and ② a "methylene blue-encapsulated silica nanoparticle-boronic acid" specifically binding to glycated hemoglobin.

Since the concentration of glycated hemoglobin in the blood sample is measured relative to the amount of total hemoglobin in the blood, in order to measure the concentration of glycated hemoglobin, the blood sample must first be hemolyzed to thus expose normal hemoglobin and glycated hemoglobin contained in the blood.

In the present invention, any hemolytic liquid may be used without limitation, as long as the hemolytic liquid has a buffer concentration at osmotic pressure at which hemolysis is feasible, and examples thereof may include TRIS, HEPES, MOPS, TES, and PIPES, each having a pH of 7 to 8.5. The blood sample that is hemolyzed includes hemoglobin and glycated hemoglobin together.

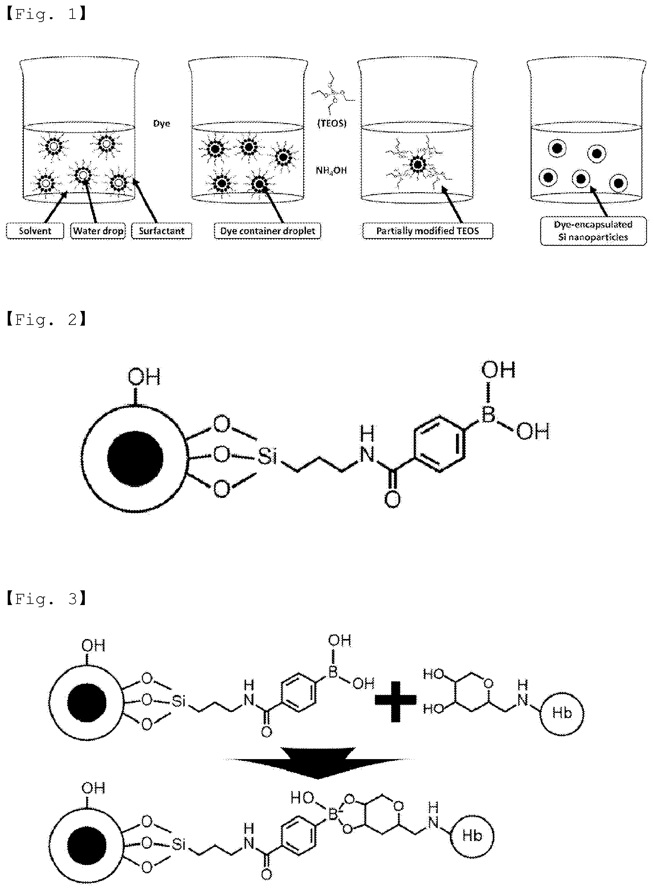

As shown in FIG. 1, the "dye-encapsulated silica nanoparticles" may be manufactured by adding a dye and silica to a mixture solution of water and a surfactant or a mixture solution of water and an organic solvent, followed by agitation and then addition of a basic catalyst.

In the present invention, the dye encapsulated in the silica nanoparticles is methylene blue having a value larger than 400 to 600 nm, within which the entire spectrum of all red hemoglobin, including hemoglobin and glycated hemoglobin, appears.

Methylated hemoglobin, which is present in a small number and which appears at 620 to 630 nm, may be excluded. The dye is more preferably methylene blue at 660 nm, which may maintain a blue color even under a basic condition of pH of 9 or more, at which the synthesis of silica nanoparticles is performed.

The surfactant is not particularly limited, but triton x-100 or n-hexane may be used in the present invention. Examples of the silica may include tetraethyl orthosilicate or tetramethyl orthosilicate.

The basic catalyst is to promote the encapsulation of the dye by a silica precursor, which may promote the hydrolysis of water and the silica precursor. The ionized silica precursors react with each other to thus produce water and alcohol (ROH), which are connected to each other to thus form a silica network and grow.

Examples of the basic catalyst may include ammonium hydroxide, tetrapropylammonium chloride, tetrapropylammonium hydroxide, tetrabutylammonium bromide, tetrabutylammonium chloride, or tetrabutylammonium hydroxide.

In the case of the "methylene blue-encapsulated silica nanoparticles", since the dye does not leak to the outside, stability and sensitivity may be increased, bio-toxicity may be low, and the functional groups on the surface thereof may be easily changed.

The diameter of the "methylene blue-encapsulated silica nanoparticles" may be 10 to 500 nm, and preferably 30 to 100 nm, which makes it possible to maintain the inherent properties of the dye. When the diameter is less than 10 nm, it is difficult to perform the operation. When the diameter is more than 500 nm, since the thickness thereof is increased, the dye may appear cloudy.

As boronic acid derivatives for imparting selectivity for glycated hemoglobin to the "dye-encapsulated silica nanoparticles", it is preferable to use 4-carboxylicphenyl boronic acid (CPBA) and 3-aminophenyl boronic acid (APBA). When the CPBA is used, the "dye-encapsulated silica nanoparticles" may be aminated. When the APBA is used, after the "dye-encapsulated silica nanoparticles" are carboxylated, conjugation may be performed via carbodiimide cross-coupling. Since the 4-carboxylicphenyl boronic acid (CPBA) has relatively higher thermal stability than the 3-aminophenyl boronic acid (APBA), it is preferable to use the CPBA.

As shown in FIGS. 2 and 3, the "dye-encapsulated silica nanoparticle-boronic acid" may react with the cis-diol of glycated hemoglobin. A plurality of dye molecules is encapsulated in the silica nanoparticles, so that the amount of light absorbed by one particle is larger than that absorbed by one dye molecule. Accordingly, the detection limit of glycated hemoglobin may be improved.

Accordingly, in another aspect, the present invention relates to a method of measuring glycated hemoglobin. The method includes (a) introducing a blood sample into a reagent composition for measuring the glycated hemoglobin, the composition including ① a hemolytic liquid and ② a "methylene blue-encapsulated silica nanoparticle-boronic acid" specifically binding to the glycated hemoglobin, followed by hemolysis and reaction, (b) injecting a reactant into an absorption pad of a cartridge, followed by washing with a washing liquid, (c) measuring an optical reflectance of the absorption pad using an optical instrument to measure the amounts of total hemoglobin and glycated hemoglobin, and (d) calculating a ratio of the glycated hemoglobin on the basis of the measured amounts of the total hemoglobin and the glycated hemoglobin.

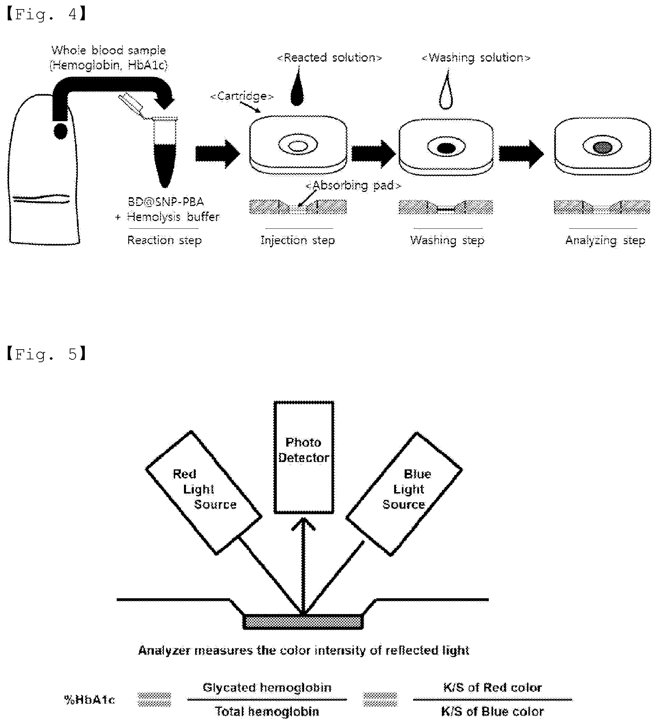

As shown in FIGS. 4 and 5, any optical instrument may be used without particular limitation as long as the optical instrument can measure optical reflectance using optical properties. The optical instrument may simultaneously radiate a wavelength for measuring total hemoglobin and a specific wavelength of the "dye(blue)-encapsulated silica nanoparticle-boronic acid" as light sources (e.g.: red (430 nm) and blue (650 to 700 nm)), and may measure the reflected optical signal using a photodiode detector (PD), thereby measuring the amounts of hemoglobin and glycated hemoglobin using an optical signal converter.

The ratio of glycated hemoglobin may be obtained by calculating the amount of glycated hemoglobin relative to the amount of total hemoglobin using the following equation.

```
Ratio (%) of glycated hemoglobin = glycated
hemoglobin / total hemoglobin
```

Generally, in the case when the ratio of glycated hemoglobin is 6.5% or more, the case may be diagnosed as diabetes.

**Mode for Invention**

Hereinafter, the present invention will be described in more detail with reference to Examples. It is to be understood by those skilled in the art that these Examples are only for illustrating the present invention and that the scope of the present invention is not to be construed as being limited by these Examples.

Example 1: Manufacture of blue dye-encapsulated silica nanoparticle-boronic acid (BD@SNP-CPBA)

1-1: Synthesis of blue dye-encapsulated silica nanoparticles (BD@SNP)

135.0 ml of cyclohexane, 31.8 ml of Triton X-100, 32.4 ml of n-hexanol, 6.12 ml of 0.1 M methylene blue, and 2.7 ml of TEOS (tetraethyl orthosilicate) were added to a 1 L round bottom flask, and uniformly mixed for 1 hour using an agitator. 1.08 ml of 25~30% aqueous ammonia ($NH_4OH$) was added thereto and reacted at room temperature for 24 hours. 200 ml of ethanol was then added to terminate the reaction. Ethanol washing and DI washing were respectively performed four times and three times using a centrifuge at 3800 rpm for 15 minutes, followed by drying in an oven at 60°C.

1-2: Amination of blue dye-encapsulated silica nanoparticles (BD@SNP)

In order to perform cross-coupling of the carboxyl groups of BD@SNP and CPBA, the hydroxyl group (-OH) on the surface of BD@SNP was substituted with a primary amine group. That is, 100 mg of BD@SNP was added to 100 ml of ethanol and dispersed for 30 minutes using an ultrasonic disperser. Then, 1 ml of APTES (3-aminopropyltriethoxysilane) was added to an agitator, followed by reaction at room temperature for 2 hours. After the reaction, ethanol washing and DI washing were respectively performed four times and three times using a centrifuge at 3800 rpm for 15 minutes, followed by drying in an oven at 60°C, thus manufacturing aminated BD@SNP (BD@SNP-$NH_2$).

1-3: Joining of aminated blue dye-encapsulated silica nanoparticles (BD@SNP-$NH_2$) and CPBA

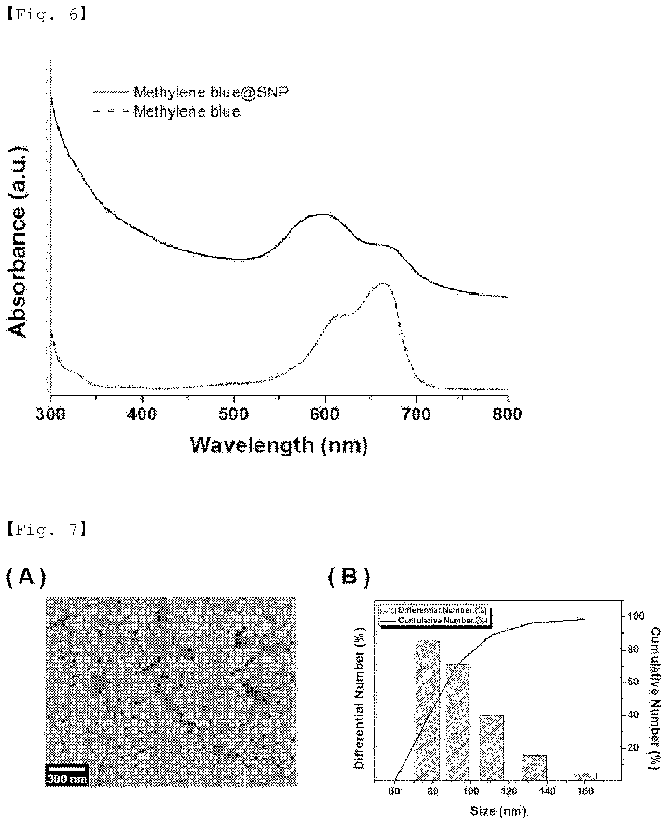

In order to provide binding ability to glycated hemoglobin, according to a carbodiimide cross-coupling method using 1-ethyl-3[3-dimethylaminopropyl]carbodiimide hydrochloride (EDC), which is a cross-coupling agent connecting a carboxyl group and a primary amine group, 4-carboxylicphenyl-boronic acid (CPBA), which is a glycated hemoglobin-binding material, was fixed on the surface of the aminated blue dye-encapsulated silica nanoparticles (BD@SNP-$NH_2$).
That is, in order to activate the carboxyl functional group of CPBA in an environment from which light was blocked, 3.48 mM CPBA was dissolved in 0.1 M MES (2-(N-morpholino)ethanesulfonic acid) buffer solution (pH 6.0), EDC having a final concentration of 1 mM was added thereto, and the reaction was allowed to progress for 30 minutes with agitation. Then, BD@SNP-$NH_2$ was added, followed by reaction in an agitator at room temperature for 10 to 20 hours.
After completion of the reaction, ethanol washing and DI washing were respectively performed four times and three times using a centrifuge at 3800 rpm for 15 minutes, followed by drying at room temperature or freeze-drying, thus manufacturing a blue dye-encapsulated silica nanoparticle-boronic acid (BD@SNP-CPBA).
Each of the "methylene blue" dye used in the synthesis and the "dye-encapsulated silica nanoparticle-boronic acid" was diluted with deionized water (DI water), and measurement with UV/Vis spectroscopy was then performed. As a result, as shown in FIG. 6, it can be seen that the "dye-encapsulated silica nanoparticle-boronic acid" has an inherent absorption wavelength of the blue dye even after the synthesis. With respect to this, the particles were analyzed using a scanning electron microscope and a dynamic light-scattering method. As a result, as shown in FIG. 7, it could be confirmed that nanoparticles having uniform morphology and a size of about 30 to 40 nm were synthesized.

Example 2: Measurement of glycated hemoglobin using a reagent composition for measuring glycated hemoglobin containing BD@SNP-CPBA and hemolytic liquid

200 μl of a reagent composition containing a hemolytic liquid ($ZnCl_2$, NaCl, $MgCl_2$, Triton X-100, $NaN_3$, glycine, HEPES, pH 8.1) and BD@SNP-CPBA manufactured in Example 1 was placed in a brown tube, and 5 μl of a blood sample in which the % value of glycated hemoglobin was measured using a reference instrument (Tosoh G11 analyzer) was added thereto, followed by reaction for 2 minutes. 25 μl of the reaction solution was put on an absorption pad of a cartridge of an optical instrument (Epithod®616, DxGen) to be absorbed for 15 seconds, and 25 μl of a washing solution (morpholine, NaCl, Triton X-100, glycerol, and $NaN_3$ mixture solution) was added thereto, followed by washing for 15 seconds. Next, the optical reflectances of red total hemoglobin and blue BD@SNP-CPBA-glycated hemoglobin on the cartridge were measured in the optical instrument (Epithod®616, DxGen). The % reflectance (%R) measured for each wavelength was converted into a K/S value, which is a quantitative index of how much of the coloring material is present on the surface thereof in use, and the formula for converting the % reflectance into the K/S value is as follows.

$$K/S = \frac{(1 - \%R)^2}{2 \times \%R}$$

(K = absorption coefficient, S = scattering coefficient)

Therefore, after the % reflectance value obtained by radiating the red light source representing the amount of glycated hemoglobin and the % reflectance value obtained from the blue light source representing the total hemoglobin were each substituted with the K/S value, the ratios thereof were calculated, thereby measuring the amount of glycated hemoglobin in the blood.

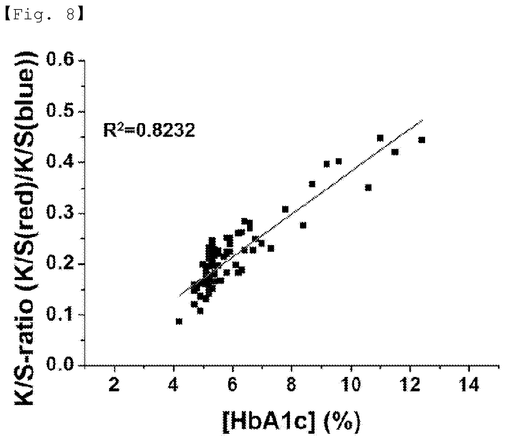

As shown in FIG. 8, it could be seen that, as the ratio of glycated hemoglobin contained in the blood is increased, the relative amount (K/S value) of the "dye-encapsulated silica nanoparticle-boronic acid" binding thereto is increased. Therefore, it was confirmed that the method of measuring glycated hemoglobin according to the present invention is capable of being widely used to diagnose diabetes.

Although specific portions of the present invention have been described in detail above, those skilled in the art will appreciate that this specific description is only a preferred embodiment, and that the scope of the present invention is not limited thereby. Accordingly, the actual scope of the present invention will be defined by the appended claims and their equivalents.

## Industrial Applicability

The reagent composition for measuring glycated hemoglobin according to the present invention contains a hemolytic liquid, so that the reagent composition may be reacted with glycated hemoglobin at the same time as hemolysis, thereby labeling glycated hemoglobin. Further, since glycated hemoglobin may be measured in a simple manner using an optical analyzer merely by injecting a washing liquid into a measurement cartridge without any separation process, the reagent composition is capable of being widely used to diagnose diabetes.

## Claims

1. A reagent composition for measuring glycated hemoglobin, the composition comprising:
① a hemolytic liquid and ② a "methylene blue-encapsulated silica nanoparticle-boronic acid" specifically binding to the glycated hemoglobin.

2. The reagent composition of claim 1, wherein the hemolytic liquid is selected from the group consisting of TRIS, HEPES, TES, MOPS, and PIPES.

3. The reagent composition of claim 1, wherein "methylene blue-encapsulated silica nanoparticles" are manufactured by adding a methylene blue and silica to a mixture solution of water and a surfactant or a mixture solution of water and an organic solvent, followed by agitation and addition of a basic catalyst, and specifically bind to the glycated hemoglobin.

4. The reagent composition of claim 1, wherein a diameter of "methylene blue-encapsulated silica nanoparticles" is 10 to 500 nm.

5. The reagent composition of claim 1, wherein the "methylene blue-encapsulated silica nanoparticle-boronic acid" is manufactured by aminating "methylene blue-encapsulated silica nanoparticles", followed by conjugation with 4-carboxylicphenyl boronic acid (CPBA), or by carboxylating the "methylene blue-encapsulated silica nanoparticles", followed by conjugation with 3-aminophenylboronic acid (APBA).

6. A method of measuring glycated hemoglobin, the method comprising:

(a) introducing a blood sample into a reagent composition for measuring the glycated hemoglobin, the composition including ① a hemolytic liquid and ② a "methylene blue-encapsulated silica nanoparticle-boronic acid" specifically binding to the glycated hemoglobin, followed by hemolysis and reaction;
(b) injecting a reactant into an absorption pad of a cartridge, followed by washing with a washing liquid;
(c) measuring an optical reflectance of the absorption pad using an optical instrument to measure amounts of total hemoglobin and the glycated hemoglobin; and
(d) calculating a ratio of the glycated hemoglobin on a basis of the measured amounts of the total hemoglobin and the glycated hemoglobin.

7. The method of claim 6, wherein the optical instrument simultaneously radiates a wavelength for measuring the total hemoglobin and a wavelength for measuring the glycated hemoglobin binding to the methylene blue-encapsulated silica nanoparticle-boronic acid as light sources, thus measuring the optical reflectance.

8. The method of claim 6, wherein diabetes is diagnosed according to the ratio of the glycated hemoglobin.

**Patentansprüche**

1. Reagenzzusammensetzung zum Messen von glykiertem Hämoglobin, wobei die Zusammensetzung umfasst:

① eine hämolytische Flüssigkeit und ② eine "Methylenblau-verkapselte Siliciumdioxid-Nanopartikel-Boronsäure", die spezifisch an das glykierte Hämoglobin bindet.

2. Reagenzzusammensetzung nach Anspruch 1, wobei die hämolytische Flüssigkeit aus der Gruppe bestehend aus TRIS, HEPES, TES, MOPS und PIPES ausgewählt ist.

3. Reagenzzusammensetzung nach Anspruch 1, wobei "Methylenblau-verkapselte Siliciumdioxid-Nanopartikel" durch Zugabe von Methylenblau und Siliciumdioxid zu einer Mischlösung aus Wasser und einem Tensid oder einer Mischlösung aus Wasser und einem organischen Lösungsmittel, gefolgt von Rühren und Zugabe eines basischen Katalysators hergestellt werden und spezifisch an das glykierte Hämoglobin binden.

4. Reagenzzusammensetzung nach Anspruch 1, wobei ein Durchmesser von "Methylenblau-verkapselten Siliciumdioxid-Nanopartikeln" 10 bis 500 nm beträgt.

5. Reagenzzusammensetzung nach Anspruch 1, wobei die "Methylenblau-verkapselte Siliciumdioxid-Nanopartikel-Boronsäure" durch Aminieren von "Methylenblau-verkapselten Siliciumdioxid-Nanopartikeln", gefolgt von Konjugation mit 4-Carboxylphenylboronsäure (CPBA) oder durch Carboxylieren der "Methylenblau-verkapselten Siliciumdioxid-Nanopartikel", gefolgt von Konjugation mit 3-Aminophenylboronsäure (APBA) hergestellt wird.

6. Verfahren zum Messen von glykiertem Hämoglobin, wobei das Verfahren umfasst:

(a) Einführen einer Blutprobe in eine Reagenzzusammensetzung zum Messen des glykierten Hämoglobins, wobei die Zusammensetzung ① eine hämolytische Flüssigkeit und ② eine "Methylenblau-verkapselte Siliciumdioxid-Nanopartikel-Boronsäure", die spezifisch an das glykierte Hämoglobin bindet, enthält, gefolgt von Hämolyse und Reaktion;
(b) Einspritzen eines Reaktionsmittels in ein Absorptionskissen einer Kartusche, gefolgt von Waschen mit einer Waschflüssigkeit;
(c) Messen eines optischen Reflexionsvermögens des Absorptionskissens unter Verwendung eines optischen Instruments, um Mengen von Gesamthämoglobin und des glykierten Hämoglobins zu messen; und
(d) Berechnen eines Verhältnisses des glykierten Hämoglobins auf der Grundlage der gemessenen Mengen des Gesamthämoglobins und des glykierten Hämoglobins.

7. Verfahren nach Anspruch 6, wobei das optische Instrument gleichzeitig eine Wellenlänge zum Messen des Gesamthämoglobins und eine Wellenlänge zum Messen des an die Methylenblau-verkapselte Siliciumdioxid-Nanopartikel-Boronsäure bindenden glykierten Hämoglobins als Lichtquellen ausstrahlt, wodurch das optische Reflexionsvermögen gemessen wird.

8. Verfahren nach Anspruch 6, wobei Diabetes gemäß dem Verhältnis des glykierten Hämoglobins diagnostiziert wird.

**Revendications**

1. Composition de réactif destinée à mesurer l'hémoglobine glyquée, la composition comportant :

① un liquide hémolytique et ② un "acide boronique à nanoparticules de silice encapsulées dans du bleu de méthylène" se liant spécifiquement à l'hémoglobine glyquée.

2. Composition de réactif selon la revendication 1, dans laquelle le liquide hémolytique est choisi parmi le groupe constitué de TRIS, HEPES, TES, MOPS et PIPES.

3. Composition de réactif selon la revendication 1, dans laquelle les "nanoparticules de silice encapsulées dans du bleu de méthylène" sont fabriquées en ajoutant un bleu de méthylène et de la silice à une solution de mélange d'eau et d'un tensioactif ou à une solution de mélange d'eau et d'un solvant organique, suivi d'une agitation et d'une addition d'un catalyseur basique, et se lient spécifiquement à l'hémoglobine glyquée.

4. Composition de réactif selon la revendication 1, dans laquelle un diamètre des "nanoparticules de silice encapsulées dans du bleu de méthylène" est de 10 à 500 nm.

5. Composition de réactif selon la revendication 1, dans laquelle "l'acide boronique à nanoparticules de silice encapsulées dans du bleu de méthylène" est fabriqué en aminant des "nanoparticules de silice encapsulées dans du bleu de méthylène", suivi d'une conjugaison avec de l'acide 4-carboxyphényl-boronique (CPBA), ou en carboxylant les "nanoparticules de silice encapsulées dans du bleu de méthylène", suivi d'une conjugaison avec de l'acide 3-aminophényl-boronique (APBA).

6. Procédé de mesure d'hémoglobine glyquée, le procédé comportant les étapes consistant à :

(a) introduire un échantillon de sang dans une composition de réactif destinée à mesurer l'hémoglobine glyquée, la composition incluant ① un liquide hémolytique et ② un "acide boronique à nanoparticules de silice encapsulées dans du bleu de méthylène" se liant spécifiquement à l'hémoglobine glyquée, suivi d'une hémolyse et d'une réaction ;
(b) injecter un réactant dans un tampon d'absorption d'une cartouche, suivi d'un lavage avec un liquide de lavage ;
(c) mesurer une réflectance optique du tampon d'absorption en utilisant un instrument optique pour mesurer des quantités d'hémoglobine totale et l'hémoglobine glyquée ; et
(d) calculer un taux de l'hémoglobine glyquée sur la base des quantités mesurées de l'hémoglobine totale et de l'hémoglobine glyquée.

7. Procédé selon la revendication 6, dans lequel l'instrument optique émet simultanément une longueur d'onde pour mesurer l'hémoglobine totale et une longueur d'onde pour mesurer l'hémoglobine glyquée se liant à l'acide boronique à nanoparticules de silice encapsulées dans du bleu de méthylène en tant que sources de lumière, en mesurant ainsi la réflectance optique.

8. Procédé selon la revendication 6, dans lequel un diabète est diagnostiqué en fonction du taux de l'hémoglobine glyquée.

【Fig. 1】

【Fig. 2】

【Fig. 3】

【Fig. 4】

Whole blood sample
(Hemoglobin, HbA1c)

<Reacted solution>

<Washing solution>

<Cartridge>

<Absorbing pad>

BD@SNP-PBA
+ Hemolysis buffer

Reaction step       Injection step       Washing step       Analyzing step

【Fig. 5】

Photo
Detector

Red
Light
Source

Blue
Light
Source

Analyzer measures the color intensity of reflected light

$$\%HbA1c \approx \frac{Glycated\ hemoglobin}{Total\ hemoglobin} \approx \frac{K/S\ of\ Red\ color}{K/S\ of\ Blue\ color}$$

【Fig. 6】

【Fig. 7】

**(A)**

**(B)**

【Fig. 8】

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5631364 A
- US 7374943 B
- WO 2014033258 A
- EP 2444803 A
- KR 1128037